# EUROPEAN PATENT APPLICATION

(11) **EP 2 199 271 A1**
(43) Date of publication of application: **23.06.2010**
(21) Application number: 08828529.1
(22) Date of filing: 28.08.2008
(51) Int. Cl.: C07C 45/63, C07C 47/575, C07C 51/363, C07C 59/56, C07C 59/64, C07C 65/03, C07C 65/21, C07C 253/30, C07C 255/54, C07B 61/00

(54) **PROCESS FOR PRODUCTION OF PHENOL DERIVATIVES SUBSTITUTED WITH IODINE AT ORTHO POSITION**

(30) Priority: 31.08.2007 JP 2007225719
(71) Applicant: Mitsubishi Gas Chemical Company, Inc., Chiyoda-ku Tokyo 100-8324 (JP)
(72) Inventor: TANAKA, Kazuo, Tokyo 100-8324 (JP)
(74) Representative: Harrison, Robert John
(86) International application number: PCT/JP2008/065403
(87) International publication number: WO 2009/028608

(57) **Abstract**

A process in which a phenol derivative is iodinated to produce a 2-iodophenol or 2,6-diiodophenol derivative substituted with iodine at an ortho position thereof is provided, which does not require any step of recovery of iodine but can produce it at low cost, in high yield and with high quality. A phenol derivative is mixed with a pyridine and hydrogen peroxide or iodic acid as an oxidizing agent, and reacted with molecular iodine. As a result, iodination can be performed very efficiently with iodine in an amount close to the theoretical amount relative to the phenol derivative, and the 2-iodophenol or 2,6-diiodophenol derivative can be obtained in high yield and with high quality.

## Description

### TECHNICAL FIELD

The present invention relates to a process for selectively producing 2-iodophenol derivatives or 2,6-diiodophenol derivatives by iodinating phenol derivatives in which one or two of the substituents at ortho positions are hydrogen. 2-iodophenol derivatives or 2,6-diiodophenol derivatives are compounds useful as raw materials for pharmaceuticals or agricultural chemicals. For instance, examples of 2-iodophenol derivatives include 2-iodo-3-hydroxy-4-methoxybenzonitrile which is an important intermediate raw material for 4-amino-6,7-dimethoxy-2-(5-methanesulfonamido-1,2,3,4-tetrahydroisoquinol-2-yl)-5-(2-pyridyl)quinazoline useful as a prostatic hyperplasia drug. Examples of 2,6-diiodophenol derivatives include 3-(3,5-diiodo-4-hydroxyphenyl)propionic acid which is an important intermediate raw material for 3,5-diiodothyropropionic acid useful as a cardiovascular or anti-hyperlipidemic drug.

### BACKGROUND ART

Conventionally, as a method for iodinating phenol derivatives, (1) a method for reacting iodine or an aqueous potassium triiodide solution with an alkali aqueous solution is generally known (for example, refer to non-Patent Document 1). Recently, (2) a method for selectively producing 2-iodo-3-hydroxy-4-methoxybenzaldehyde by iodinating 3-hydroxy-4-methoxybenzaldehyde with iodine monochloride has been reported (for example, refer to non-Patent Document 2). Also, (3) a method for selectively producing 3-(3,5-diiodo-4-hydroxyphenyl)propionic acid by reacting 3-(4-hydroxyphenyl)propionic acid with iodine, potassium iodide and methylamine (for example, refer to Patent Document 2).

(1) In the method of the non-Patent Document 1, iodine is introduced in ortho or para position relative to the phenolic hydroxyl group so that the resulting product may be a mixture of 4-iodophenol derivatives, 2-iodophenol derivatives and 2,4-diiodophenol derivatives, and generally there is no case where a single component is selectively obtained.
(2) In the method of the non-Patent Document 2, 3-hydroxy-4-methoxybenzaldehyde is reacted with iodine monochloride in pyridine and dioxane at room temperature for 6 days to effect selective iodination at an ortho position relative to the hydroxyl group, yielding 2-iodo-3-hydroxy-4-methoxybenzaldehyde at a yield of 73%. However, this has a problem in industrial practice since iodine monochloride is difficult to handle, and the reaction takes several days to provide a sufficient yield.
(3) The method of the Patent Document 1 is a process for selectively producing 3-(3,5-diiodo-4-hydroxyphenyl)propionic acid in which iodine is charged in an amount at least double the theoretical amount relative to 3-(4-hydroxyphenyl)propionic acid. Therefore, only a half amount of iodine is used for 3-(3,5-diiodo-4-hydroxyphenyl)propionic acid, and steps for collecting and re-using the remaining iodine are required, thereby causing problems such that the steps become complicated and costly. Also, since a great amount of an intermediate 3-(3-iodo-4-hydroxyphenyl)propionic acid is by-produced, load of purification is increased. (4) On the other hand, there have been cases where nuclear iodination of aromatic compounds using iodine and hydrogen peroxide has been applied to iodination of biphenyl (for example, refer to Patent Document 2), but no case has been disclosed where it is applied to phenol derivatives.
   Patent Document 1: JP-A-2004-522747
   Patent-Document 2: JP-A-S63-91336
   Non-Patent Document 1: New Experimental Chemistry Course (Shin-Jikken Kagaku Koza) (Maruzen Company Limited, published in 1977), Synthesis and reaction of organic compounds (Yuuki-kagoubutsu no gosei to hannou) (I), Vol. 14, p. 423.
   Non-Patent Document 2: K. M. Markovich, V. Tantishaiyakul, A. Hamada, D. D. Miller, K. J. Romstedt, G. Shams, Y. Shin, P. F. Fraundorfer, K. Doyle, and D. R. Feller, J. Med. Chem. 1992, 35, 466-479

### DISCLOSURE OF THE INVENTION

### PROBLEM TO BE SOLVED BY THE INVENTION

The present invention aims at solving the above problem of the conventional techniques and providing a process in which a 2-iodophenol or 2,6-diiodophenol derivative which is selectively iodinated at an ortho position thereof is obtained by iodinating a phenol derivative in which one or two of the substituents at the ortho positions are hydrogen, without need of steps for recovery of iodine, at low cost, in high yield and with high quality.

### MEANS FOR SOLVING THE PROBLEM

As a result of diligent researches for solving the above mentioned problem, the present inventor has found that when a 2-iodophenol or 2,6-diiodophenol derivative represented by the general formula (2) is obtained from a phenol derivative in which one or two of the substituents at the ortho positions are hydrogen as represented by the general formula (1), iodination can be performed very efficiently with iodine in an amount close to a theoretical amount relative to the phenol derivative as a raw material by effecting the reaction using molecular iodine in a presence of a pyridine and an oxidizing agent. Also, the present inventor has found that the aimed 2-iodophenol or 2,6-diiodophenol derivative can be produced in high yield and with high quality, and thus has completed the present invention.

That is, as shown in the following items (1)-(9), the present invention relates to a process for producing a 2-iodophenol or 2,6-diiodophenol derivative represented by the general formula (2) by allowing a phenol derivative in which one or two of the substituents at the ortho positions relative to the hydroxyl group are hydrogen as represented by the general formula (1) to react with molecular iodine in a presence of an oxidizing agent and a pyridine.
(1) A process for producing a phenol derivative substituted with iodine at an ortho position, wherein a phenol derivative in which one or two of the substituents at the ortho positions relative to the hydroxyl group are hydrogen as represented by the general formula (1) is iodinated with molecular iodine in a presence of an oxidizing agent and a pyridine, to yield a 2-iodophenol or 2,6-diiodophenol derivative represented by the general formula (2). 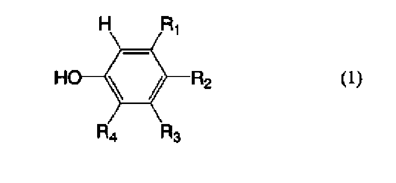 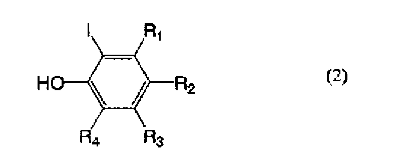 wherein R₁, R₂, R₃ and R₄ of the formula (1) or (2) are independent from each other, R₁ represents a hydrogen atom, formyl group or cyano group, R₂ represents a hydrogen atom, formyl group, cyano group, methoxy group or group represented by (CH₂)COOH with n = 0 to 5, R₃ represents a hydrogen atom, formyl group or cyano group and R₄ represents a hydrogen atom, iodine atom or methoxy group, excluding the case where R₁, R₂, R₃ and R₄ are all hydrogen atoms.)
(2) The process for producing a phenol derivative substituted with iodine at an ortho position, according to item (1), wherein the oxidizing agent is one or more selected from the group consisting of hydrogen peroxide and iodic acid.
(3) The process for producing a phenol derivative substituted with iodine at an ortho position, according to item (2), wherein a ratio of an amount to be used of hydrogen peroxide relative to molecular iodine is 0.6 to 4.5 on molar basis.
(4) The process for producing a phenol derivative substituted with iodine at an ortho position, according to item (2), wherein a ratio of an amount to be used of iodic acid relative to molecular iodine is 0.01 to 1.5 on molar basis.
(5) The process for producing a phenol derivative substituted with iodine at an ortho position, according any one of items (1) to (4), wherein the pyridine is one or more selected from the group consisting of pyridine, methylpyridine, dimethylpyridine and quinoline.
(6) The process for producing a phenol derivative substituted with iodine at an ortho position, according to any one of items (1) to (5), wherein a ratio of an amount to be used of the pyridine relative to the phenol derivative represented by the general formula (1) is 0.1 to 20 on molar basis.
(7) The process for producing a phenol derivative substituted with iodine at an ortho position, according to any one of items (1) to (6), wherein 3-hydroxy-4-methoxybenzaldehyde is iodinated to yield 2-iodo-3-hydroxy-4-methoxybenzaldehyde.
(8) The process for producing a phenol derivative substituted with iodine at an ortho position, according to any one of items (1) to (6), wherein 3-hydroxy-4-methoxybenzonitrile is iodinated to yield 2-iodo-3-hydroxy-4-methoxybenzonitrile.
(9) The process for producing a phenol derivative substituted with iodine at an ortho position, according to any one of items (1) to (6), wherein 3-(4-hydroxyphneyl)propionic acid is iodinated to yield 3-(4-hydroxy-3,5-diiodophenyl)propionic acid.

### EFFECT OF THE INVENTION

According to the present invention, a 2-iodophenol or 2,6-diiodophenol derivative which is substituted with iodine at an ortho position and useful as a pharmaceutical intermediate, can be produced at low cost, in high yield and with high quality.

### BEST MODE FOR CARRYING OUT THE INVENTION

The phenol derivative used as a raw material in the present reaction is one in which one or two of the substituents at the ortho positions relative to the hydroxyl group are hydrogen as represented by the general formula (1) wherein R₁, R₂, R₃ and R₄ are independent from each other, R₁ represents a hydrogen atom, formyl group or cyano group, R₂ represents a hydrogen atom, formyl group, cyano group, methoxy group or group represented by (CH₂)ₙCOOH with n = 0 to 5, R₃ represents a hydrogen atom, formyl group or cyano group, and R₄ represents a hydrogen atom, iodine atom or methoxy group, excluding the case where R₁, R₂, R₃ and R₄ are all hydrogen atoms. Concrete examples of the compounds represented by the general formula (1) include 3-hydroxy-4-methoxybenzaldehyde, 3-hydroxy-4-methoxybenzonitrile, 4-hydroxybenzoic acid, 3-methoxy-4-hydroxybenzoic acid, 2-(4-hydroxyphenyl)acetic acid and 3-(4-hydroxyphenyl)propionic acid, and particularly preferable examples thereof include 3-hydroxy-4-methoxybenzaldehyde, 3-hydroxy-4-methoxybenzonitrile and 3-(4-hydroxyphenyl)propionic acid.

Examples of the pyridine used in the present reaction include pyridine, chloropyridine, methylpyridine, dimethylpyridine, ethylpyridine, ethylmethylpyridine, bipyridyl, quinoline, isoquinoline, quinoline carboxylic acid and dimethylquinoline. Preferable is pyridine, methylpyridine, dimethylpyridine and quinoline. Referring to the amount to be used, pyridines are used preferably in an amount of 0.1 to 20 times the phenol derivative as a raw material on molar basis, and are used more preferably in an amount of 0.2 to 10 times on molar basis. When the amount of the pyridine is less than 0.1 times on molar basis, iodinated compounds may not be obtained depending upon a substrate to be used. On the other hand, the upper limit is not particularly limited, but when it exceeds 20 times on molar basis, a large amount of the pyridine must be recovered after the reaction, and thus it is not economical.

The solvent used in the present reaction may be one which is inactive to the present reaction and may be in an amount sufficient for the reaction mixture to be always stirred. When a pyridine is used as a solvent, they may be used singly or as a mixed solvent with an organic solvent such as acetonitrile or another pyridine, or may contain water. The blending ratio thereof may be a ratio that allows the phenol derivative as a substrate and molecular iodine to sufficiently dissolve therein.

The reaction temperature is preferably 10-100°C, and more preferably 20-70°C. Even when the reaction temperature is lower than this, the reaction may proceed, but a sufficient reaction rate may not be obtained, and when it is higher than this, side reaction such as generation of high boiling point products increases unfavorably. The reaction time is suitably in a range of 1 to 5 hours.

The oxidizing agent used in the present invention is preferably hydrogen peroxide and iodic acid. Hydrogen peroxide may be in a form of an ordinary aqueous hydrogen peroxide. Referring to the concentration thereof, a high concentration of not less than 60% is not particularly required, but a concentration of 30-60% which is usually handled in the industry is sufficient. The amount of hydrogen peroxide is preferably 0.6 to 4.5 times the molecular iodine on molar basis, and more preferably 0.8 to 4 times on molar basis. When it is less than 0.6 times on molar basis, a sufficient reaction rate or yield may not be obtained, and when it exceeds 4.5 times on molar basis, there is no problem in reaction rate, but decrease of selectivity due to side reaction becomes large unfavorably. Besides, hydrogen peroxide may be wholly added from the beginning of the reaction to the reaction system, or may be consecutively added thereto as the reaction progresses, and from the viewpoint of the reaction efficiency, the consecutive addition is preferable. Also, oxidizing agents other than hydrogen peroxide may be used including iodic acid, perchloric acid, perchloric acid salts such as sodium perchlorate and potassium perchlorate, periodic acid, periodic acid salts such as sodium periodate and potassium periodate and persulfuric acid salts such as sodium persulfate, and they may be used in combination as required to the extent that does not impair the object of the present invention.

The amount to be used of the iodic acid is 0.01 to 1.5 times the molecular iodine on molar basis in terms of weight ratio. The iodic acid is solid, and may be used for the reaction as it is or may be used in a form of a solution or suspension in an appropriate solvent.

The amount of the molecular iodine used in the present invention may be a theoretical amount relative to the substrate. When a 2-iodophenol derivative is synthesized, it may be 0.3 to 0.7 times the phenol derivative on molar basis, and 0.5 times that is the theoretical amount is preferable. When a 2,6-diiodophenol derivative is synthesized, it may be 0.6 to 1.4 times the phenol derivative on molar basis, and one times that is the theoretical amount is preferable. When the amount of the molecular iodine to be used is less than these ranges, conversion of the phenol derivative becomes low, and productivity may be lowered, so that unreacted phenol derivative may remain, thereby requiring purification of the resulting 2-iodophenol or 2,6-diiodophenol derivative. When it exceeds these ranges, the target compound is further iodinated to produce an iodinated high boiling point product. The molecular iodine may be wholly added from the beginning of the reaction to the reaction system, or may be consecutively added thereto as the reaction progresses. The molecular iodine does not have to completely dissolve from the beginning of the reaction.

The method of the present invention is high in selectivity of the target 2-iodophenol derivatives or 2,6-diiodophenol derivatives, and thus can provide highly-purified 2-iodophenol derivatives or 2,6-diiodophenol derivatives in high yield by only adding water to the solution resulting from the iodination so as to precipitate the product. The amount of water to be added depends on the concentration of the substrate to be charged, and may be usually an amount not more than 10 parts by weight relative to 1 part by weight of the reacted solution.

Although iodine or hydrogen peroxide may sometime remain in the reacted solution, precipitation of iodine crystals or detoxification of hydrogen peroxide can be performed by previously adding a reducing agent such as sodium sulfite, sodium hydrogen sulfite or sodium thiosulfate to the reacted solution. The addition amount of sodium sulfite, sodium hydrogen sulfite or sodium thiosulfate is preferably 1 to 5 times the total of the iodine and hydrogen peroxide remaining after the completion of the reaction on molar basis, more preferably 1.2 to 3 times on molar basis.

When the phenol derivative has a carboxyl group, namely, is a hydroxyphenyl carboxylic acid derivative, it may form a pyridinium salt with a pyridine, but in this instance, it can be crystallized as a hydroxyiodophenyl carboxylic acid derivative by acid-precipitation with an aqueous solution of an inorganic acid such as hydrochloric acid or an organic acid such as acetic acid. The precipitated crystals are collected by filtration. Also, in order to further improve crystal purity of the resulting 2-iodophenol or 2,6-diiodophenol derivative, purification by recrystallization or other treatments may be performed.

### EXAMPLE

Hereinafter, the present invention will be described in more detail by way of Examples and Comparative Examples. However, the present invention is in no way restricted to these Examples.

### Example 1

A mixture of 1.66 g (10 mmol) of 3-(4-hydroxyphenyl)propionic acid, 3 g (38 mmol) of pyridine and 2.54 g (10 mmol) of molecular iodine was maintained at 40°C, and 2.5 g (22 mmol as hydrogen peroxide) of a 30% aqueous hydrogen peroxide was dropped thereto over 0.4 hour. After the completion of addition of the aqueous hydrogen peroxide, the reaction solution was maintained at 40°C, and stirred for 1.6 hour. Then, 5 g of a 20% aqueous sodium sulfite solution was added thereto. At room temperature, the reaction solution was added little by little to 20 g of a 2N aqueous hydrochloric acid solution under stirring. After the whole amount of the reaction solution was added thereto, 60 g of water was added thereto. The precipitated crystals were separated by filtration and vacuum dried at 70°C to obtain 3.9 g of crystals. According to analyses of the crystals and the mother liquid with high performance liquid chromatography, conversion of 3-(4-hydroxyphenyl)propionic acid was 100%, selectivity of 3-(4-hydroxy-3,5diiodophenyl)propionic acid was 94% on both substrate basis and iodine basis, selectivity of 3-(4-hydroxy-3iodophenyl)propionic acid was 0.4%, isolated yield of 3-(4-hydroxy-3,5diiodophenyl)propionic acid was 93%, and crystal purity of 3-(4-hydroxy-3,5diiodophenyl)propionic acid was 99%.

### Example 2

A mixed solution of 1.66 g (10 mmol) of 3-(4-hydroxyphenyl)propionic acid, 1.54 g (6 mmol) of molecular iodine and 3 g (38 mmol) of pyridine was stirred at 40°C, and 2.9 g (8 mmol as iodic acid) of a 70% aqueous iodic acid solution was dropped thereto over 0.5 hour. After the completion of addition of the aqueous iodic acid solution, the reaction was continued at 40°C for 1.5 hours. After the reaction was terminated, 5 g of a 20% aqueous sodium sulfite solution was added thereto. Then, the reaction solution was added little by little to 20 g of a 2N aqueous hydrochloric acid solution under stirring at room temperature, and then 60 g of water was added thereto. The precipitated crystals were separated by filtration and vacuum dried at 70°C to obtain 3.90 g of crystals. According to analyses of the crystals and the mother liquid with high performance liquid chromatography, reaction yield of 3-(4-hydroxy-3,5diiodophenyl)propionic acid was 90% on both substrate basis and iodine basis, reaction yield of 3-(4-hydroxy-3iodophenyl)propionic acid was 7%, isolated yield of 3-(4-hydroxy-3,5diiodophenyl)propionic acid crystals was 89%, and purity of 3-(4-hydroxy-3,5diiodophenyl)propionic acid was 95%.

### Comparative Example 1

According to the method disclosed in JP-A-2004-522747, the experiment was reproduced. A mixed solution of 0.16 g (1 mmol) of 3-(4-hydroxyphenyl)propionic acid, 0.53 g (2 mmol) of molecular iodine, 0.93 g (12 mmol as CH₃NH₂) of a 40% aqueous methylamine solution and 1.1 g of water was stirred at 40°C, and a mixed solution of 0.53 g (2 mmol) of molecular iodine, KI (0.55 g, 3 mmol) and 1.4 g of water was added little by little over 10 minutes, and reaction was effected at 23°C for 5 hours. After the completion of the reaction, a 2N aqueous hydrochloric acid solution was added to the reaction solution to precipitate crystals. The suspension was filtered, dried and analyzed. Conversion of 3-(4-hydroxyphenyl)propionic acid was 100%, selectivity of 3-(4-hydroxy-3,5diiodophenyl)propionic acid was 89% on substrate basis, and selectivity of 3-(4-hydroxy-3,5diiodophenyl)propionic acid was 40% on iodine basis. Also, crystal purity of 3-(4-hydroxy-3,5diiodophenyl)propionic acid was 93%, and crystal isolated yield thereof was 89%.

### Example 3

A mixed solution of 0.345 g (2.1 mmol) of 4-hydroxy-3-methoxybenzoic acid, 0.27 g (1.05 mmol) of molecular iodine and 0.45 g (5.7 mmol) of pyridine was stirred at 40°C, and 0.26 g (2.31 mmol as hydrogen peroxide) of a 30% aqueous hydrogen peroxide solution was dropped thereto over 0.4 hour. After the completion of addition of the aqueous hydrogen peroxide, the reaction solution was maintained at 40°C, and stirred for 1.6 hours. The total reaction time was 2 hours. Then, 1.0 g of a 20% aqueous sodium sulfite solution was added thereto. At room temperature, the reaction solution was added little by little to 4 g of a 2N aqueous hydrochloric acid solution under stirring. After the whole amount of the reaction solution was added thereto, 15 g of water was added thereto. The precipitated crystals were separated by filtration and vacuum dried at 70°C to obtain 0.525 g of crystals. According to analyses of the crystals and the mother liquid with high performance liquid chromatography, conversion of 4-hydroxy-3-methoxybenzoic acid was 100%, selectivity of 4-hydroxy-3-methoxy-5-iodobenzoic acid was 93% on both substrate basis and iodine basis, isolated yield of 4-hydroxy-3-methoxy-5-iodobenzoic acid was 87%, and crystal purity thereof was 99.7%.

### Example 4

A mixture of 1.38 g (10 mmol) of 4-hydroxybenzoic acid, 3.94 g (50 mmol) of pyridine and 2.67 g (10 mmol) of molecular iodine was maintained at 40°C, and 2.5 g (23.6 mmol as hydrogen peroxide) of a 30% aqueous hydrogen peroxide was dropped thereto over 0.4 hour. After the completion of addition of the aqueous hydrogen peroxide, the reaction solution was maintained at 40°C, and stirred for 1.6 hours. Then, 5 g of a 20% aqueous sodium sulfite solution was added thereto. At room temperature, the reaction solution was added little by little to 20 g of a 2N aqueous hydrochloric acid solution under stirring. After the whole amount of the reaction solution was added thereto, 60 g of water was added thereto. The precipitated crystals were separated by filtration and vacuum dried at 70°C to obtain 3.24 g of crystals. According to analyses of the crystals and the mother liquid with high performance liquid chromatography, conversion of 4-hydroxybenzoic acid was 88.7%, selectivity of 4-hydroxy-3,5-diiodobenzoic acid was 29.0% on 4-hydroxybenzoic acid substrate basis, and selectivity of 4-hydroxy-3-iodobenzoic acid was 27.0%.

### Example 5

A mixture of 4.53 g (30 mmol) of 3-hydroxy-4-methoxybenzonitrile with a purity of 98.8%, 4.32 g (55 mmol) of pyridine and 3.81 g (15 mmol) of molecular iodine was maintained at 40°C, and 4.98 g (43.9 mmol) of a 30% aqueous hydrogen peroxide was dropped thereto over an hour. After the completion of addition of the 30% aqueous hydrogen peroxide, the reaction solution was maintained at 40°C, and stirred for an hour. Then, 45 g of a 10% aqueous sodium sulfite solution was added thereto, maintained at 40°C for 0.5 hour, and left to cool to room temperature. The precipitated crystals were separated by filtration and vacuum dried at 70°C to obtain 7.92 g of crystals. According to analyses of the crystals and the mother liquid with gas chromatography, conversion of 3-hydroxy-4-methoxybenzonitrile was 97.3%, selectivity of 2-iodo-3-hydroxy-4-methoxybenzonitrile was 95.1%, selectivity of 2,6-diiodo-3-hydroxy-4-methoxybenzonitrile was 3.2%, isolated yield of 2-iodo-3-hydroxy-4-methoxybenzonitrile was 91.7%, and crystal purity of 2-iodo-3-hydroxy-4-methoxybenzonitrile was 95.5%. Meanwhile, yields of 2-iodo-3-hydroxy-4-methoxybenzonitrile on 3-hydroxy-4-methoxybenzonitrile basis and iodine basis were both 92.5%

### Example 6

A mixture of 4.56 g (30 mmol) of 3-hydroxy-4-methoxybenzaldehyde, 4.36 g (55 mmol) of pyridine and 3.81 g (15 mmol) of molecular iodine was maintained at 30°C, and 4.98 g (43.9 mmol) of a 30% aqueous hydrogen peroxide was dropped thereto over an hour. After the completion of addition of the 30% aqueous hydrogen peroxide, the reaction solution was maintained at 30°C, and stirred for 2 hours. Then, 45 g of a 10% aqueous sodium sulfite solution was added thereto, maintained at 40°C for 0.5 hour and left to cool to room temperature. The precipitated crystals were separated by filtration and vacuum dried at 70°C to obtain 6.54 g of crystals. According to analyses of the crystals and the mother liquid with gas chromatography, conversion of 3-hydroxy-4-methoxybenzaldehyde was 93.0%, selectivity of 2-iodo-3-hydroxy-4-methoxybenzaldehyde was 94.3%, selectivity of 6-iodo-3-hydroxy-4-methoxybenzaldehyde was 1.5%, isolated yield of 2-iodo-3-hydroxy-4-methoxybenzaldehyde was 75.0%, and crystal purity of 2-iodo-3-hydroxy-4-methoxybenzaldehyde was 95.7%. Meanwhile, yields of 2-iodo-3-hydroxy-4-methoxybenzaldehyde on 3-hydroxy-4-methoxybenzaldehyde basis and iodine basis were both 87.7%.

### Examples 7-8 and Comparative Example 2

In Example 1, the ratio of pyridine to 3-(4-hydroxyphenyl)propionic acid was changed to study the effect of pyridine. Meanwhile, acetonitrile was added in place of pyridine so that the concentrations of 3-(4-hydroxyphenyl)propionic acid, iodine and hydrogen peroxide were the same as in Example 1. In the other respects, experiments were performed in the same manner as in Example 1. The results are shown in Table 1.

**Table 1**

| Example | Pyridine/substrate (molar ratio) | Conversion (%) | Yield (%) | |
|---|---|---|---|---|
| | | | 2,6-diiodo form | 2-iodo form |
| Example 1 | 3.8 | 100 | 93 | 0.4 |
| Example 7 | 1.9 | 100 | 89 | 2 |
| Example 8 | 0.95 | 100 | 70 | 21 |
| Comparative Example 2 | 0 | 100 | 38 | 52 |

### INDUSTRIAL APPLICABILITY

The present invention can be utilized for production of 2-iodophenol derivatives or 2,6-diiodophenol derivatives which are compounds useful as raw materials for pharmaceuticals and agricultural chemicals.

## Claims

1. A process for producing a phenol derivative substituted with iodine at an ortho position, wherein a phenol derivative in which one or two of the substituents at the ortho positions relative to the hydroxyl group are hydrogen as represented by the general formula (1) is iodinated with molecular iodine in a presence of an oxidizing agents and a pyridine, to yield a 2-iodophenol or 2,6-diiodophenol derivative represented by the general formula (2). 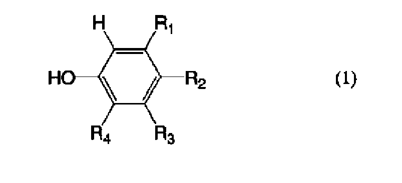 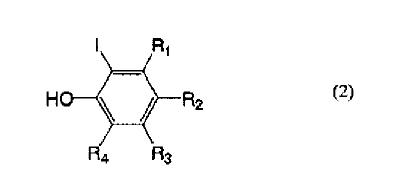 wherein R₁, R₂, R₃ and R₄ of the formula (1) or (2) are independent from each other, R₁ represents a hydrogen atom, formyl group or cyano group, R₂ represents a hydrogen atom, formyl group, cyano group, methoxy group or group represented by (CH₂)ₙCOOH with n = 0 to 5, R₃ represents a hydrogen atom, formyl group or cyano group, and R₄ represents a hydrogen atom, iodine atom or methoxy group, excluding the case where R₁, R₂, R₃ and R₄ are all hydrogen atoms.)

2. The process for producing a phenol derivative substituted with iodine at an ortho position, according to claim 1, wherein the oxidizing agent is one or more selected from the group consisting of hydrogen peroxide and iodic acid.

3. The process for producing a phenol derivative substituted with iodine at an ortho position, according to claim 2, wherein a ratio of an amount to be used of hydrogen peroxide relative to molecular iodine is 0.6 to 4.5 on molar basis.

4. The process for producing a phenol derivative substituted with iodine at an ortho position, according to claim 2, wherein a ratio of an amount to be used of iodic acid relative to molecular iodine is 0.01 to 1.5 on molar basis.

5. The process for producing a phenol derivative substituted with iodine at an ortho position, according any one of claims 1 to 4, wherein the pyridine is one or more selected from the group consisting of pyridine, methylpyridine, dimethylpyridine and quinoline.

6. The process for producing a phenol derivative substituted with iodine at an ortho position, according to any one of claims 1 to 5, wherein a ratio of an amount to be used of the pyridine relative to the phenol derivative represented by the general formula (1) is 0.1 to 20 on molar basis.

7. The process for producing a phenol derivative substituted with iodine at an ortho position, according to any one of claims 1 to 6, wherein 3-hydroxy-4-methoxybenzaldehyde is iodinated to yield 2-iodo-3-hydroxy-4-methoxybenzaldehyde.

8. The process for producing a phenol derivative substituted with iodine at an ortho position, according to any one of claims 1 to 6, wherein 3-hydroxy-4-methoxybenzonitrile is iodinated to yield 2-iodo-3-hydroxy-4-methoxybenzonitrile.

9. The process for producing a phenol derivative substituted with iodine at an ortho position, according to any one of claims 1 to 6, wherein 3-(4-hydroxyphenyl)propionic acid is iodinated to yield 3-(4-hydroxy-3,5-diiodophenyl)propionic acid.
